# EUROPEAN PATENT APPLICATION

(11) **EP 2 823 761 A1**
(43) Date of publication of application: **14.01.2015**
(21) Application number: 13757310.1
(22) Date of filing: 08.03.2013
(51) Int. Cl.: A61B 5/11

(54) **DEVICE AND METHOD FOR EVALUATING FUNCTIONAL CAPACITY**

(30) Priority: 09.03.2012 ES 201230353
(71) Applicant: Universidad de Zaragoza, 50009 Zaragoza (ES)
(72) Inventor: Marín Zurdo, José Javier, E-50009 Zaragoza (ES); Auría Apilluelo, José Manuel, E-50009Zaragoza (ES); Huertas Talón, José Luis, E-50009 Zaragoza (ES)
(74) Representative: Illescas, Manuel
(86) International application number: PCT/ES2013/070145
(87) International publication number: WO 2013/132129

(57) **Abstract**

The present invention relates to a device and a method for the functional capacity evaluation of subjects, applicable in different environments of the biomedical, biosanitary or labour type. More specifically, the invention proposed by the applicant relates to a device and to a method based on objective techniques for measuring the movements and efforts made by the subjects under evaluation, such that it is possible to achieve an accurately and reproducibly analysis of the physical ability of the subjects to perform certain activities of daily living or in the course of their work activity. Preferably, the invention relates to the functional evaluation of the upper limbs of the human body, as well as the spine.

## Description

### FIELD OF THE INVENTION

The present invention relates to a device and a method for the functional capacity evaluation of subjects, applicable in different environments of the biomedical, biosanitary or labour type. More specifically, the invention proposed by the applicant relates to a device and to a method based on objective techniques for measuring the movements and efforts made by the subjects under evaluation, such that it is possible to achieve an accurately and reproducibly analysis of the physical ability of the subjects to perform certain activities of daily living or in the course of their work activity. Preferably, the invention relates to the functional evaluation of the upper limbs of the human body, as well as the spine.

### BACKGROUND OF THE INVENTION

At present, evaluation of functional capacity (commonly referred to as Functional Capacity Evaluation or FCE) is applicable in many fields of human activity in which physical movements or efforts are made, such as in disease diagnosis, medico-legal report writing, rehabilitation of patients with musculoskeletal ailments or diseases, or occupational risk assessment and prevention, among others.

At diagnostic and/or forensic level, FCE may be used, for example, as a tool for evaluating physical injury due to trauma or diseases in patients, or for determining the existence of physical limitations in a subject and quantification thereof, in an objective and reproducible manner. The evaluation of the consequences of traffic accidents, where permanent injuries constitute partial or total incapacity for the victim's regular occupation or activity is especially relevant in this field.

In rehabilitation treatments, FCE makes it possible to determine the efficiency of said treatments, on facilitating the evaluation of the subjects at different stages thereof.

In the labour environment, in accordance with the type of work and specific needs of the tasks to be performed by the subjects under evaluation, FCE makes it possible to determine the subjects' capacity to perform said work in healthy conditions, in addition to analyse and predict the work potential of a certain subject, establishing a sufficient safety margin to reduce the possibility of injuries or relapses, on establishing the restrictions that must be taken into account when assigning said subject to a certain workplace.

In the state of the art corresponding to the field of the present invention, mention should be made of the existence of biomechanical analysis devices such as that disclosed in patent application PCT WO 9713454 A1 (1997), which describes integrated movement analysis systems based on surface electromyography techniques (i.e. recording of the electrical activity produced by the skeletal muscles of the subjects under evaluation using electrode systems) together with means for performing dynamometric analysis of a range of movements, together with other functional capacity evaluation tests for monitoring the functional capacity of musculoskeletal groups of the human body.

While the aforementioned devices represent an accurate approximation for the recording of the electrical activity produced by the skeletal muscles of the subjects under evaluation, they are not entirely effective for recording the accuracy of the skeletal movement thereof, due to the inaccuracy of the mechanical systems they include for such purpose, based on cervical collars, harnesses and manipulable levers, whose behaviour is extremely dependent on the physical characteristics of the subjects, which produces substantial differences in behaviour between different evaluations, with the ensuing loss of accuracy and reproducibility in the results obtained.

With the aim of remedying the problems arising from the lack of accuracy on evaluating the movement of the previously described devices, new FCE systems based on computer-assisted motion capture and photogrammetry (i.e. determination of geometric properties from the captured images). Said computer-assisted motion capture techniques are known in the technical field of the invention and an example thereof is patent application US 2002/0116990 A1 (2002), which discloses a system for analysing the movement of subjects, preferably destined for analysing neck movement, comprising means for analysing the movement of the head or body of said subjects, by means of the use of reflective markers placed on their shoulders and head, together with a plurality of cameras for analysing the movement of the musculoskeletal groups under evaluation. The system also includes a computer for processing the data obtained.

The systems cited in the preceding paragraph, while representing a significant improvement in accuracy as regards motion analysis based on dynamometry and electromyography techniques, also have the drawback that the results obtained using said techniques are heavily dependent on the behaviour of the subject under evaluation, or of the instructions received by the person in charge of the evaluation, even in a single subject, as it is possible that from one test to another the range of movements or postures is modified. Therefore, even if said movement could be measured accurately, it implies a source of uncertainty in the results and scarce reproducibility thereof.

Therefore, it is necessary to develop new FCE devices and methods that will solve the aforementioned problems by means of tools that will improve the conditions in which the evaluation procedures are performed and that, likewise, will limit the dependence between the results obtained and the physical characteristics of the subjects under evaluation.

The present invention is aimed at meeting said need.

### BRIEF DESCRIPTION OF THE INVENTION.

Therefore, the present invention is aimed at the development of new tools destined for evaluating the functional capacity of subjects that will improve the tools currently available in the state of the art. Said objective is achieved, in a first aspect of the invention, by means of a device comprising:
- a support-bearing structure configured to sustain one or more supports disposed at adjustable spatial heights or orientations by means of fixation means for fixing the supports to said support-bearing structure, wherein said structure houses the elements with which the subject under evaluation may interact during the evaluation and during which his or her movements are analysed.
- one or more multifunctional elements disposed in the support-bearing structure by means of fixations to the supports, wherein said elements are defined as means configured to house manipulable objects with which the subject may interact during the evaluation. Therefore, by means of the programmed manipulation of the objects by the subject, the evaluators may more accurately analyse his or her movements and efforts, using the complementary techniques available in the state of the art. The use of the device of the invention makes it possible, on enabling adjustment of the height and orientation of the supports on which the subject acts, to fully adapt the specific conditions thereof, and provides a stable reference for conducting the tests, significantly improving the results obtained in the evaluation.

In an embodiment of the invention, one or more of the multifunctional elements comprised in the device are passive elements, i.e. acting as static supports or containers of the manipulable objects, and the subject may move the aforementioned objects from one multifunctional element to another of the device.

In another embodiment of the invention, one or more of the multifunctional elements are active elements, i.e. they are configured as levers, push-buttons, controls, knobs and/or buttons with which the subject may interact.

In an additional embodiment of the invention, one or more of the multifunctional elements of the device comprise activable visual indicators, configured to provide a visual reference for the subject. By means of the programmed activation of said indicators, it is possible to plan the movements to be made by the subject during the evaluation, with the corresponding increase in the accuracy of the analysis thereof. Preferably, the visual indicators are luminous indicators.

In a preferred embodiment of the invention, the device comprises means for controlling, for example, a computer, configured to activate and/or deactivate the visual indicators. This makes it possible to automatically record each activation sequence, together with the movements to be made by the subject.

In another embodiment of the invention, the device comprises a motion capture system for capturing the three-dimensional movement of the subject under evaluation based on inertial sensors and/or photogrammetry; and/or a system for capturing the muscle activity log of the subject under evaluation by means of surface electromyography and/or measuring the external efforts made on manipulating the objects by means of dynamometry. This provides an adequate means for correctly analysing the movement and/or efforts made by the subject under evaluation during the test, added to the advantages provided by the support technology proposed by the present invention.

In an additional embodiment, the device control means is configured to control the motion capture system for capturing the three-dimensional movement of the subject under evaluation and/or the system for capturing the muscle activity log of said subject. This makes it possible to centralise the analysis of the subject's movement and/or efforts and to programme the movements to be performed, thereby achieving an improvement in the accuracy of the analysis and in the reproducibility of each evaluation.

In another embodiment of the invention, the objects manipulable by the subject have different shapes, masses, sizes or textures. This makes it possible to provide a complementary means for studying movement under different configurations of the manipulable objects.

In a preferred embodiment of the invention, the support-bearing structure of the device comprises at least one substantially vertical rod standing on a base. This makes it possible to achieve a stable structure that is, in turn, easy to manage and configure.

In an additional embodiment of the invention, the device comprises a plurality of rods disposed in the manner of a front and/or side casing around the subject. This makes it possible to evaluate wide-ranging movement of both the arms and the dorso-cervical region by, for example, moving an object from a multifunctional element disposed on one side of the subject to another element disposed on the opposite side thereof.

In another embodiment of the invention, the spatial arrangement of the device is variable, in such a manner that the spatial positions and/or orientations of the support-bearing structure and/or of its associated supports may be adjusted. This confers greater configurability to the invention.

Another purpose of the present invention relates to a method for obtaining data useful in the evaluation of the functional capacity of a subject, which comprises the use of a device according to any of the embodiments of the invention. Said method comprises, preferably, studying the subject while he or she manipulates or moves, at least, one manipulable object on, from or to a certain multi-functional element, said element being indicated through the activation of a visual indicator.

In a preferred embodiment of the method of the invention, the following steps are carried out:
- Lighting of one of the visual indicators associated with a multifunctional element.
- Manipulation or movement by the subject under evaluation of a manipulable object associated with the multifunctional element.
- Recording of the movement and/or muscle activity of the subject under evaluation while he or she manipulates or moves the manipulable object.

In another embodiment of the invention, the evaluation method comprises the activation and deactivation of visual indicators that mark one or more multifunctional elements for distracting the subject. This gives rise to a tool that makes it possible to measure the subject's adaptability to the tests conducted, together with the analysis of the surprise movements thereof.

In a preferred embodiment of the method of the invention, the musculoskeletal activity of the subject under evaluation is recorded by control means configured to control a three-dimensional motion capture system using inertial sensors and/or photogrammetry, in order to control a system for capturing the muscle activity log of the subject under evaluation using surface electromyography or dynamometry, and/or for controlling the activation or deactivation of visual indicators. This makes it possible to obtain an accurate analysis of the movements and efforts made by the subject.

In another embodiment of the invention, the method comprises limiting the subject's field of vision during evaluation thereof. This makes it possible to enhance the performance of full, wide-ranging movements of the spine by the subject under evaluation.

The method of the invention is specially designed as a tool for studying the spine or upper limb of the subject under evaluation.

In addition to those already designed, other characteristics and advantages of the invention will be conferred from the following description, as well as the drawings attached hereto.

### DESCRIPTION OF THE DRAWINGS

Figure 1 shows a perspective view of an embodiment of the device of the invention.
Figure 2 shows a perspective view of an embodiment of the device of the invention based on passive multifunctional elements.
Figure 3 shows a perspective view of an embodiment of the device of the invention based on active multifunctional elements.
Figure 4 shows a perspective view of an embodiment of the device of the invention based on a support-bearing structure comprising multiple rods and wherein the multifunctional elements are passive.
Figure 5 shows a perspective view of an embodiment of the invention based on a support-bearing structure comprising multiple rods and wherein the multifunctional elements are both active and passive.
Figure 6 shows a view of the device of the invention, during its use in a human model to which a plurality of motion and surface electromyography sensors have been applied, in order to record the motion and muscle activity of the subject under evaluation.

### DETAILED DESCRIPTION OF THE INVENTION

The present invention relates to a device and to a method for the functional capacity evaluation of subjects, applicable in different environments of the biomedical, biosanitary or labour type.

More specifically, the invention proposed relates to a device and to a method based on objective techniques for motion and effort measurement, thereby enabling the accurate and reproducible evaluation of a subject's functional capacity, i.e. his or her physical ability to perform activities of daily living or labor tasks. Preferably, the invention relates to the functional capacity evaluation of the upper limbs of the human body, as well as the spine.

The device of the invention is designed as a system that complements the technical tools available in the state of the art for evaluating the motion and muscle activity of a subject. Said techniques comprise, mainly, three-dimensional (3D) motion capture based, preferably, on inertial motion sensors (combination of magnetometers, inclinometers and accelerometers along the three axes of space) and/or photogrammetry (i.e. the determination of geometric properties from images) and recording of the patient's muscle activity, by means of electromyography (recording of electrical activity of human skeletal muscles) or dynamometry.

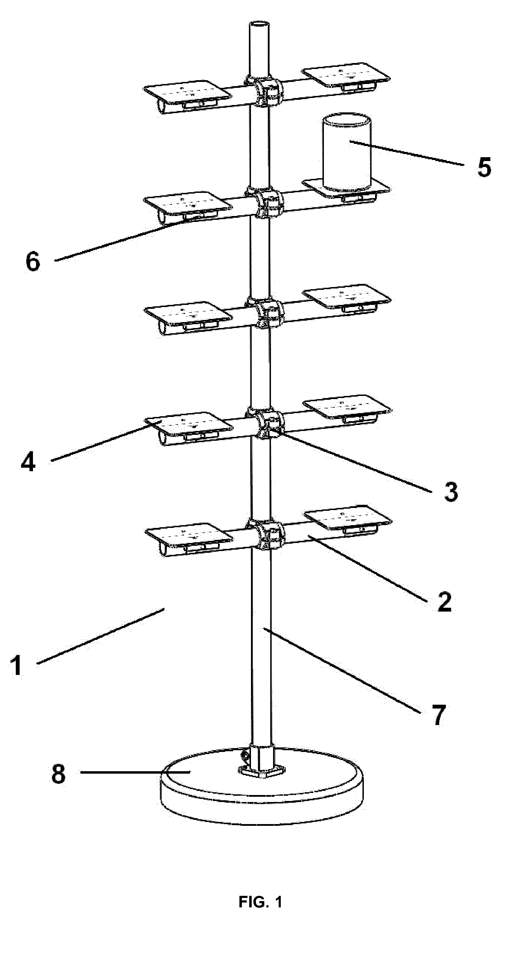

In this context, this device is constituted as a system that can be used in combination with the aforementioned techniques, so that the measurements obtained for the functional evaluation are more accurate, reproducible and independent of the specific characteristics of the subject under evaluation. To such end, as shown in figure 1 herein, the device of the invention comprises a support-bearing structure (1) destined for sustaining one or more supports (2) at a certain height above the ground. Said supports may be directly integrated in the support-bearing structure (1), or fixed thereto using adequate fixation means (3), such as clamps, screws or any other means known in the state of the art for such purpose. In a preferred embodiment of the invention, the fixation means (3) are adjustable, so that the height at which the supports are fixed (2) or their spatial orientation are variable, thereby enabling their adaptation to the conditions of each subject.

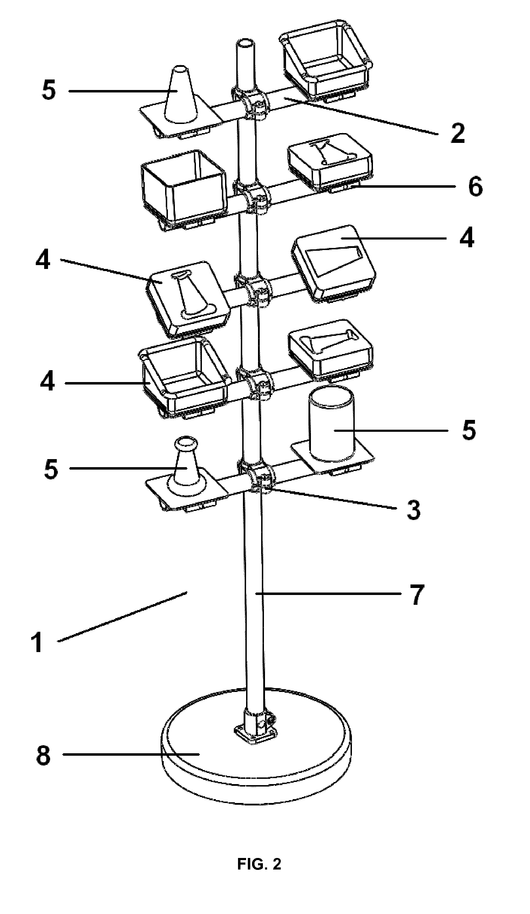

At least one multifunctional element (4) is fixed to each of the supports (2), where said element is defined as a means configured to house manipulable objects (5) with which the subject may interact during the evaluation. The multifunctional elements (4) may be, in different embodiments of the invention, both active and passive elements. When their role is passive, they act as static supports or containers of the manipulable objects (5), and the subject can move the aforementioned objects (5) from one multifunctional element (4) to another, in the embodiments of the invention that comprise a plurality of said elements (4). An example of a device according to the invention, based on passive multifunctional elements (4), is shown in figure 1. In said figure, the manipulable objects (5) are cylinders destined for being moved by the subject under evaluation. Figure 2 shows another embodiment of the invention based on passive multifunctional elements (4), where said elements comprise support trays, boxes or housing means adapted to the shapes of the objects manipulable (5) by the subject under evaluation.

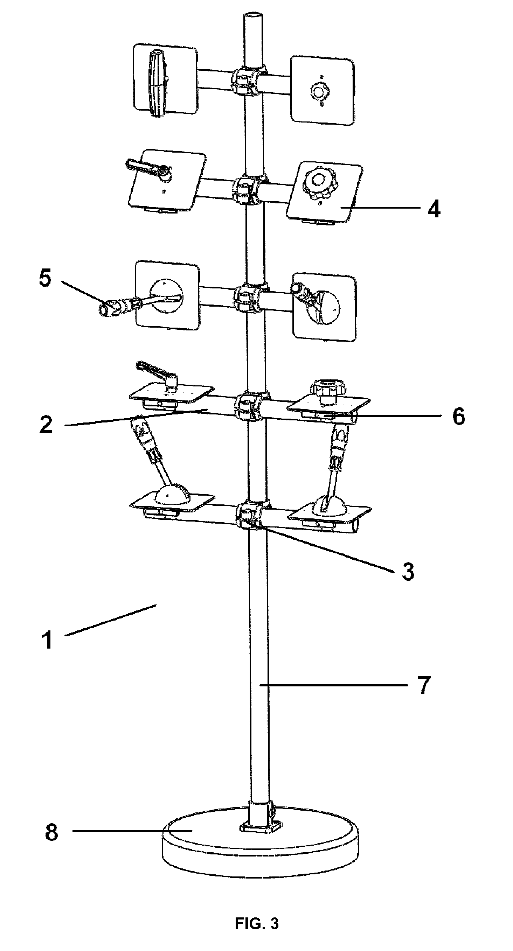

When the role of the multifunctional elements (4) is active, the manipulable objects (5) that house said elements are configured so that the subject may perform certain actions or manipulations thereon, such as, for example, turns, pulsations, pressures, etc. In the embodiments of the invention in which some of the multifunctional elements (4) are active, the manipulable objects (5) may be controls, levers, wheels, buttons or other equivalent elements capable of performing a similar function. An embodiment of the invention based on active multifunctional elements (4) is shown in figure 3 herein.

In a preferred embodiment of the invention, the multifunctional elements (4) have activable visual indicators (6) that provide a visual reference for the subject, in such a manner as to allow the person in charge of the evaluation to direct the actions of the subject under evaluation through the programmed activation of said visual indicators (6) and following a sequence of movements previously established in the functional capacity evaluation method used. Preferably, the visual indicators (6) are luminous indicators, for example, light-emitting diodes (LEDs).

In different embodiments of the invention, the manipulable objects (5) may adopt different shapes (cylindrical, prismatic or other) and different masses and/or textures, as can be observed in figure 2. In these embodiments, the corresponding multifunctional elements (4) are of the passive type and many adopt different configurations, both in shape (to adapt to the manipulable objects (5), and in spatial orientation, such that the subject needs to adopt different postures of the wrist (flexion-extension or lateralization) and forearm (pronation or supination) shoulder (flexion, abduction and/or rotation) when gripping, depositing or handling the manipulable objects (5). Said types of movement are important in the evaluation of the functional capacity of the upper limb.

In other embodiments, a single evaluation device may comprise a combination of both active and passive multifunctional elements (4) for the purpose of analysing a broad spectrum of functional movements in the subject under evaluation, as shown in figure 3.

Through the use of the functional evaluation device, the movement and effort made by the patient may be monitored, using the aforementioned tools of the state of the art (3D motion capture, dynamometry, electromyography, etc.), in relation to preset references (i.e. the height of the supports, which may be adjusted by the evaluators), thereby obtaining a more accurate assessment than would be obtained without the existence of said reference points. In this regard, the present invention represents a major improvement over conventional functional capacity evaluation techniques.

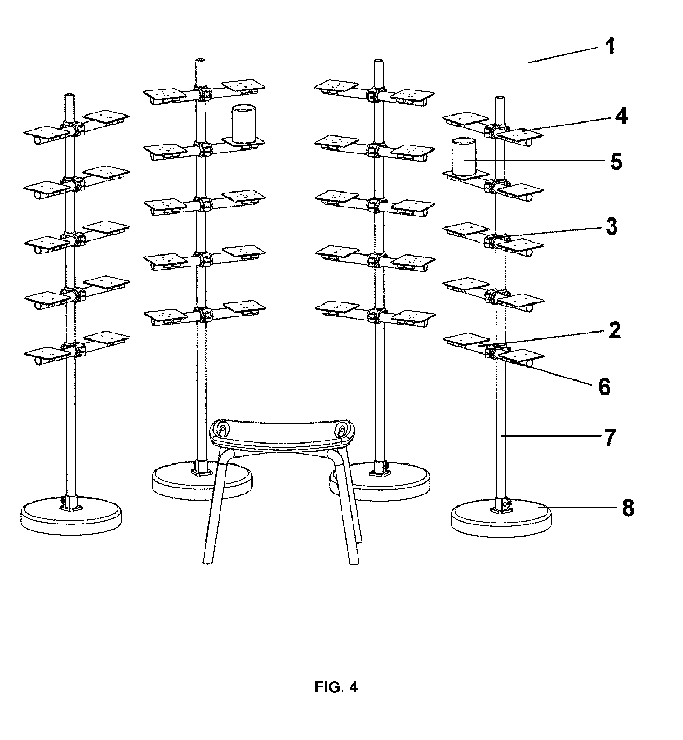

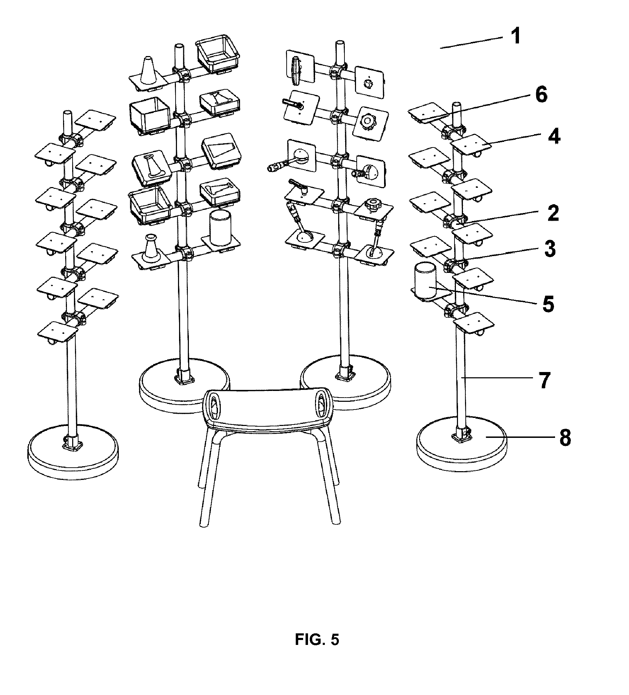

While figures 1 to 3 herein show support-bearing structures (1) based on a plurality of supports (2) fixed to a single, substantially vertical rod (7) standing on a base (8), in another embodiment of the present invention it is possible to combine two or more rods (7) disposed in the manner of a front and/or lateral casing around the subject under evaluation, as shown, for example, in figures 4 to 5. Through this embodiment, it is possible to evaluate wide-ranging movements of both the arms and the dorso-cervical region by, for example, moving a manipulable object (5) from one multifunctional element (4) disposed on one side of the subject, to another element (4) disposed on the opposite side thereof. Likewise, in an embodiment of the invention comprising visual indicators (6) applied to a support-bearing structure (1) of multiple rods (7) (Figures 4 to 5), it is possible to evaluate the functional capacity of the back and neck, through the location by the subject under evaluation of a certain multifunctional element (4) indicated by the visual indicator (6). This makes it possible to direct the attention and subsequent movements of the subject towards different regions of the space that encompass the support-bearing structures (1), enabling the accurate evaluation of preprogrammed movements and the inclusion of distraction actions aimed at making the subject move his or her neck, in a nearly unconscious manner, to locate the visual indicator (6) activated at a given time.

The spatial arrangement of the functional evaluation device is, preferably, configurable, making it possible to adjust the spatial positions of the support-bearing structure (1) and its associated supports (2), and enabling the adaptation of the device of the invention to the physical characteristics of each subject. It is also possible to configure the typology, arrangement and number of multifunctional elements (4) distributed in each support, and the shape and weight of the manipulable objects (5) in accordance with the different functional variables/aspects to be evaluated in the subject.

In addition, the different configurations of the device make it possible to conduct tests in which the subject is both sitting and standing. In those cases in which the study is performed sitting, it is possible to incorporate a chair adjustable in height to the device, integrated and adapted to the geometry of the support-bearing structure (1).

Both the means for analysing the motion and muscle activity of the subject, and the process for activating and deactivating the visual indicators (6) of the present evaluation device are preferably controlled by a computer, which acts as a control means for the device and makes it possible to define and implement various evaluation routines or procedures, recording the results obtained in the study of each subject.

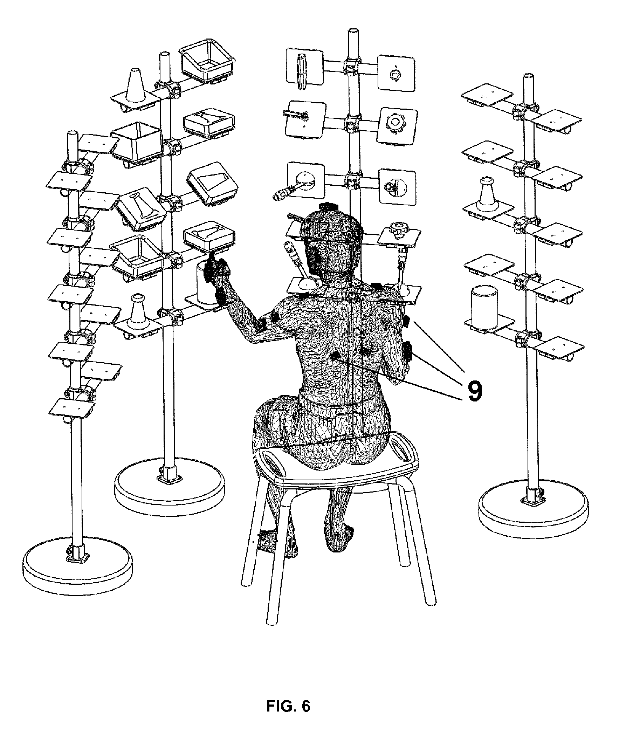

Another object of the present invention relates to a method for evaluating functional capacity. Said method is based on the programming of the movements made by the subject in accordance with a predefined protocol, using the evaluation device described in the preceding paragraphs. In this manner, the persons in charge of the evaluation give the subject instructions for performing a set of actions aimed at the movement and manipulation of manipulable objects (5) in different multifunctional elements (4) of the device, during which, preferably, the movement of the subjects, and of the muscle activity involved, is analysed by means of motion capture tools. Both analyses are performed through the installation of movement and/or electromyography sensors on the body segments of the subject under evaluation, as shown in figure 6. Therefore, through the use of different protocols, it is possible to evaluate the functional capacity of the dorsal lumbar and cervical spine, shoulders, elbows and/or wrists. Once the data resulting from the measuring equipment have been processed by the computer, a functional capacity evaluation report of the subject is generated, in accordance with the anatomical aspects and areas to be studied. It is also possible to include, within the protocol used, unexpected instructions for the subject following various distraction strategies.

The evaluation method proposed is also characterised in that it involves the performance of various tests which, adequately combined in each case, make it possible to measure certain movement and effort parameters, several times, in different ways or situations and at different instants, making it possible to study the consistency or inconsistency thereof and, consequently, to detect possible simulations or distortions generated by the subject, determining whether said subject has collaborated adequately or not during the evaluation. The method of the invention also helps to establish a classification based on the loss of functional capacity of a person who has suffered some type of injury, whether in the upper limbs or spine.

In an embodiment of the method of the invention, the subject commences in a starting position with arms pressed against the body and elbows bent, and must move manipulable objects (5), of a specific weight, from one set of multifunctional elements (4) to another, following a predefined sequence. Each of the multifunctional elements (4) will have a visual indicator (6) for the purpose of indicating the following element where the subject must position the manipulable object (5). The sequence in which the indicators will light up is controlled, preferably, by a computer programmed with software and/or hardware developed for such purpose.

In the various embodiments of the invention that comprise the evaluation of the functional capacity of the two arms, the subject is requested to perform alternating actions, which he or she must perform using his or her right or left hand, but in such a manner that the set of movements made using either arm are the same at the end of the test.

In each evaluation, once the subject is situated in the starting position, the evaluator commences the test, which starts when one of the visual indicators (6) associated with a multifunctional element (4) lights up. At that instant, the subject must identify it. If it is an active multifunctional element (4), he or she must perform the corresponding action (turn, press or pull, as the case may be); if it is a passive multifunctional element (4), he or she must move a manipulable object (5) from one position to the position corresponding to the multifunctional element (4) indicated by, for example, a glowing visual indicator (6), and deposit the object in the orientation corresponding to said passive multifunctional element (4). Next, the subject returns to his or her initial resting position and at that instant the evaluator ends this movement and initiates a new one until concluding the set of movements that compose the sequence.

Figure 6 shows a view of the device of the invention during its use in a human model to whom a plurality of movement and/or surface electromyography sensors (9) have been applied, in order to record the movement and/or muscle activity of the subject under evaluation.

In order to force the patient to make wide-ranging head movements, the field of vision of the person under evaluation is preferably limited by applying a means for limiting the field of vision, preferably an opaque screen attached to the patient's head and with a limited field of vision, in such a manner that all the subjects under evaluation have the same field of vision during the tests. The distance between the eyes and the screen will allow the subject to wear his or her own glasses where necessary. This limitation of the field of vision forces the subject to perform wide-ranging cervical movements. The subsequent analysis thereof is decisive for the evaluation of his or her cervical spine.

Several sets of movements can be performed in order to establish comparisons between them, interspersing a rest period adequate to the requirements of the test.

In the different embodiments of the invention, the time used to perform the tests, the maximum amplitudes achieved by the joints involved, the presence of possible compensations and the speed of execution thereof are measured. Likewise, the electromyographic activity of the muscles involved in the test is also analysed.

Lastly, the advantages it provides over the current functional analysis tools available in the market are worth briefly repeating below:
- Objectiveness of the measurements made, which allows a more rigorous quantification of the injury or of its consequences.
- Capacity for reproducibility or repeatability.
- It combines different techniques: analysis of the articular movement, bioelectrical analysis of the associated muscle activity or measurement of efforts with the use of dynamometers.
- Introduce distraction techniques that favour active and relaxed participation of the subject during the test, facilitating an evaluation not influenced by patients with a deceptive or non-collaborative behaviour.
- It is a method open to incorporating new measuring instruments and new technologies, such as virtual reality using, inter alia, 3D glasses and movement or compression gloves.

Having described the present invention and some of its preferred embodiments, together with its main advantages over the state of the art, it is worth repeating the fact that its application must not be understood as being necessarily limited to a certain configuration of the elements of the device or to the method described, or to the embodiments referred to in the examples of the invention, but rather that it is also applicable to other types of configurations and procedures, by means of the adequate variations in its elements, provided that said variations do not alter the essence of the invention and object thereof.

## Claims

1. Device for evaluating the functional capacity of a subject, **characterised in that** it comprises:
- a support-bearing structure (1) configured to sustain one or more supports (2) disposed at adjustable heights or spatial orientations using fixing means (3) for fixing said supports (2) to said support-bearing structure (1);
- one or more multifunctional elements (4) disposed on the support-bearing structure (1) by means of fixations to the supports (2), where said multifunctional elements (4) are configured to house one or more objects manipulable (5) by the subject under evaluation.

2. Device, according to the preceding claim, wherein one or more of the multifunctional elements (4) are passive elements, configured as supports or containers of the manipulable objects (5).

3. Device, according to any of the preceding claims, wherein one or more of the multifunctional elements (4) are active elements, configured as levers, push-buttons, controls, knobs and/or buttons.

4. Device, according to any of the preceding claims, wherein one or more of the multifunctional elements (4) comprise activable visual indicators (6), configured to provide a visual reference for the subject.

5. Device, according to the preceding claim, wherein the visual indicators (6) are luminous indicators.

6. Device, according to any of claims 4 to 5, comprising control means configured to activate and/or deactivate the visual indicators (6).

7. Device, according to any of the preceding claims, comprising a motion capture system for capturing the three-dimensional movement of the subject under evaluation comprising inertial sensors and/or photogrammetry, and/or a system for capturing the muscle activity log of the subject under evaluation using surface electromyography and/or measuring the external forces exerted by the subject during manipulation of the objects using dynamometry.

8. Device, according to the preceding claim, wherein the control means is configured to control the motion capture system for capturing the three-dimensional movement of the subject under evaluation and/or the system for capturing the muscle activity log of said subject.

9. Device, according to any of the preceding claims, wherein the manipulable objects (5) comprise different shapes, masses, sizes or textures.

10. Device, according to any of the preceding claims, wherein the support-bearing structure (1) comprises at least one substantially vertical rod (7) standing on a base (8).

11. Device, according to the preceding claim, comprising a plurality of rods (7) disposed as a front and/or side casing around the subject under evaluation.

12. Device, according to any of the preceding claims, **characterised in that** its spatial arrangement is variable, in such a manner that the spatial positions and/or orientations of the support-bearing structure (1) and/or of its associated supports (2) may be adjusted.

13. Method for obtaining useful data in the evaluation of the functional capacity of a subject, comprising the use of a device according to any of the preceding claims.

14. Method, according to the preceding claim, comprising the study of the subject while he or she manipulates or moves, at least, one manipulable object (5) on, from or to a certain multifunctional element (4), said element being indicated by means of the activation of a visual indicator (6).

15. Method, according to claims 13 to 14 comprising the following steps:
- lighting of one of the visual indicators (6) associated with a multifunctional element (4);
- manipulation or movement by the subject under evaluation of a manipulable object (5) associated with the multifunctional element (4);
- recording of the movement and/or muscle activity of the subject under evaluation while he or she manipulates or moves the manipulable object (5).

16. Method, according to claims 13 to 15, comprising the activation and deactivation of visual indicators (6) that indicate one or more multifunctional elements (4) for distracting the subject.

17. Method, according to any of claims 14 to 16, comprising the recording of the movement and/or muscle activity of the subject under evaluation through control means which is configured for controlling a three-dimensional motion capture system constituted by inertial sensors and/or photogrammetry, to control a system for capturing the muscle activity log of the subject under evaluation by means of surface electromyography or dynamometry, and/or for controlling-the activation or deactivation of visual indicators (6).

18. Method, according to any of claims 13 to 17, comprising the limitation of the subject's field of vision during his or her evaluation.

19. Method, according to any of claims 13 to 18, comprising the study of the spine or of the upper limb of the subject under evaluation.
